Europäisches Patentamt

European Patent Office

Office européen des brevets

(19)

(11) Veröffentlichungsnummer : **0 212 192**
**B1**

(12) **EUROPÄISCHE PATENTSCHRIFT**

(45) Veröffentlichungstag der Patentschrift :
13.12.89

(51) Int. Cl.⁴ : **A 61 F   2/30**

(21) Anmeldenummer : 86109228.6

(22) Anmeldetag : 07.07.86

(54) Endoprothese zum Ersatz des Mittelabschnitts eines langgestreckten Knochens.

(30) Priorität : 09.08.85 DE 3528728

(43) Veröffentlichungstag der Anmeldung :
04.03.87 Patentblatt 87/10

(45) Bekanntmachung des Hinweises auf die Patenterteilung : 13.12.89 Patentblatt 89/50

(84) Benannte Vertragsstaaten :
DE FR GB IT SE

(56) Entgegenhaltungen :
DE–A– 2 049 111
DE–A– 3 138 848
DE–A– 3 205 577
GB–A– 2 137 098
GB–A– 2 137 884
US–A– 4 502 160

(73) Patentinhaber : Waldemar Link GmbH & Co
Barkhausenweg 10
D-2000 Hamburg 63 (DE)

(72) Erfinder : Keller, Arnold
An der Naherfurth 5
D-2061 Kayhude (DE)

(74) Vertreter : Glawe, Delfs, Moll & Partner Patentanwälte
Postfach 26 01 62 Liebherrstrasse 20
D-8000 München 26 (DE)

**Beschreibung**

Bei ausgedehnten Knochendefekten, insbesondere bei tumorbedingten Resektionen, besteht die Möglichkeit, durch eine Distanzprothese befallene Gliedmaßen, in weitgehend normaler Funktion zu erhalten bzw. eine sonst manchmal notwendige Amputation zu vermeiden.

Zur Befestigung solcher Distanzprothesen, insbesondere im Mittelabschnitt von Röhrenknochen, ist vorzugsweise der Markkanal, proximal und distal, des verbleibenden Knochens geeignet. Dafür muß allerdings nach proximal bzw. distal ein marknagelähnlicher Stift eingebracht werden.

Weichteil-, muskel- und sehnenbedingt ist eine entsprechend weite Extension nicht möglich. Aus diesem Grund werden bereits bekannte Distanzprothesen zweigeteilt verwendet, d. h. der eine Teil wird erst distal eingebracht, der andere Teil proximal und dann in der Mitte zusammengefügt. Bei dieser Form der Distanzprothese ist nachteilig, daß die mittlere Befestigung durch Schrauben etc. in ihrer Stabilität als nicht optimal anzusehen ist. Ein weiterer Nachteil besteht darin, daß diese Prothesen vorher exakt in der notwendigen Größe ausgemessen und vorhanden sein müssen, und kaum die Möglichkeit eines feinjustierbaren Längenausgleiches beinhalten.

Eine weitere Form von bekannten Distanzstücken besteht aus einem Rohr, welches dem Maß der erforderlichen Distanz entsprechen soll. Dieses Rohr wird während der Operation zwischen den verbleibenden Knochenabschnitten, distal und proximal, gehalten, während man vom äußeren proximalen bzw. distalen Ende des betroffenen Gliedmaßes einen Nagel einschlägt, der das rohrförmige Distanzstück auffädelt und damit fixiert. Diese Methode erfordert eine ausgedehntere Operation und erfordert gleichzeitig nachteilig den Zugang zu der Einschlagstelle für den Nagel. Bei bestimmten Gliedmaßen, z. B. Vorderarm bzw. der Tibia (Unterschenkel), ist ein derartiges Vorgehen fast unmöglich. Die Erfindung bezieht sich daher auf eine Endoprothese zum Ersatz des Mittelabschnitts eines langgestreckten Knochens, insbesondere eines Röhrenknochens, die aus einem hülsenförmigen Distanzstück und einem darin längs verschiebbaren Nagel zum Zusammenwirken mit einem Knochenendstück besteht.

Die der Erfindung zugrundeliegende Aufgabe besteht in der Schaffung einer solchen Endoprothese, die operationstechnisch einfacher und mit geringerer Traumatisierung einsetzbar ist.

Die erfindungsgemäße Lösung besteht darin, daß der Nagel einschließlich seines mit dem Knochenendstück zusammenzuwirken bestimmten Abschnitts in dem Distanzstück aufnehmbar und von der Seite des Distanzstücks her verschiebbar ist.

Zweckmäßigerweise ist ein Knochennagel mit einem Ende des Distanzstücks fest verbunden, während lediglich der für das andere Ende vorgesehene Knochennagel in dem Distanzstück verschiebbar ist. In diesem Fall kann die Prothese durch Abwinkeln der distal bzw. proximal verbleibenden Knochenenden eingebracht werden, indem zunächst der fest mit dem Distanzstück verbundene Knochennagel in ein Ende eingetrieben wird. Danach werden beide verbleibenden Knochenteile in Funktionsstellung ausgerichtet, wobei das Distanzstück der Prothese zwischen den beiden verbleibenden Knochenteilen positioniert ist. Durch Einfügen von Unterlegscheiben läßt sich nun die erforderliche Distanz problemlos herstellen. Danach wird der in dem Distanzstück befindliche zweite Nagel in den anderen Knochenabschnitt eingetrieben.

Dafür ist in dem Distanzstück ein Schlitz vorhanden, durch den das zweite Nagelende mit einem geeigneten Instrument eingetrieben werden kann. Nach erfolgtem Eintreiben des zweiten Nagelendes, wird dieses durch eine aus dem Schlitz des Distanzstückes herausragende Befestigungsschraube fixiert. Weil das zweite Nagelende keinerlei Druckbelastung ausgesetzt ist, kommt dieser Fixation eine untergeordnete Bedeutung zu. Neben der Möglichkeit durch Unterlegen von Unterlegscheiben die notwendige Distanzlänge der Prothese während der Operation passend herzustellen, besteht eine weitere vorteilhafte Variante des feindosierbaren Längenausgleichs bei der Prothese durch ein mit links und rechts gewindeversehenes Mittelstück im Bereich des zum Distanzausgleich vorgesehenen Teils der Prothese. Mit diesem Zwischenstück (Muffe) ist eine weitere Distanzdosierbarkeit gegeben und ermöglicht darüber hinaus, falls erforderlich, eine Extension bei bereits geschrumpften Muskelverhältnissen.

Um das zweite Nagelende durch den Schlitz austreiben zu können, ist die mit Links- und Rechtsgewinde versehene Muffe ebenfalls geschlitzt. Dieser Schlitz muß zum Austreiben des zweiten Nagels mit dem Schlitz des Distanzersatzes in Deckung gebracht werden. Während des Distanzausgleiches mit der Muffe des Distanzstückes werden die beiden in den Knochen eingebrachten Nagelenden des Distanzstückes keinen Rotationskräften unterworfen, so daß die durch das Einschlagen entstandene Verankerung nicht gefährdet wird.

Zur zementlosen Verankerung der Nagelenden haben diese vorzugsweise ein im Querschnitt sternförmiges Profil, welches in der Längsrichtung gleichförmig ist. Ebenfalls für die zementierte Verankerung sind die Nagelenden in Längsrichtung profiliert, um eine Rotationsstabilität zu gewährleisten.

Die Erfindung wird im folgenden näher unter Bezugnahme auf die Zeichnungen erläutert. Darin zeigen :

Fig. 1 u. 2 eine Seitenansicht und einen Längsschnitt durch eine erste Ausführungsform der Prothese,

Fig. 3 bis 6 eine zweite Ausführungsform in Seitenansicht, im Längsschnitt im Ausgangszustand bzw. in situ und schließlich im Querschnitt,

Fig. 7 bis 12 den Operationsvorgang schematisch unter Verwendung einer Prothese gemäß Fig. 1 und 2.

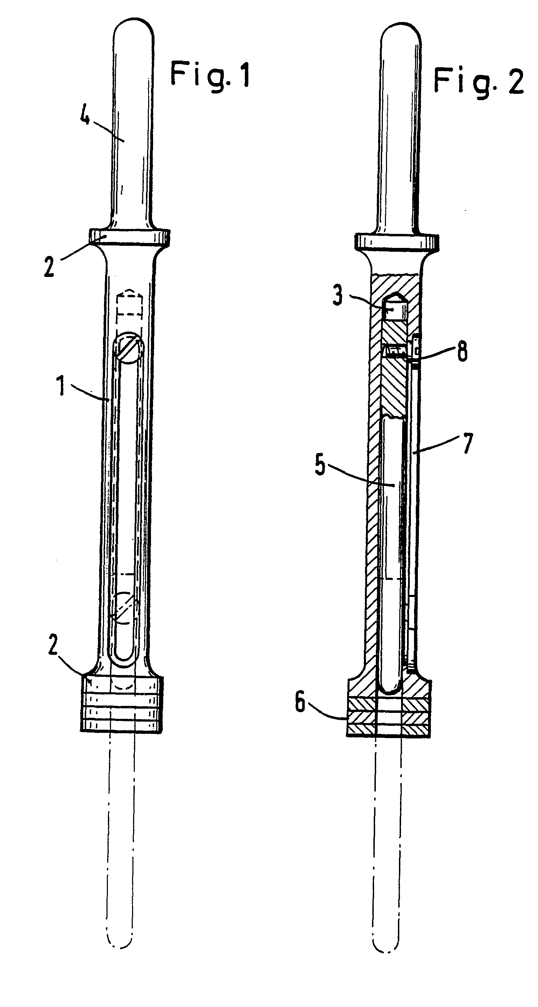

Die Prothese gemäß Fig. 1 und 2 besteht aus dem Distanzstück 1, das zweckmäßigerweise verbreitete Auflageteller 2 bildet und eine Längsbohrung 3 enthält, die an einem Ende offen ist. Das andere Ende trägt, fest mit dem Distanzstück 1 verbunden, ein Nagelende 4. Die Bohrung 3 enthält den anderen Nagel 5, der in Fig. 2 in der Ausgangsstellung mit durchgehenden Linien innerhalb des Distanzstücks 1 und mit strichpunktierten Linien im vorgesehenen Endzustand dargestellt ist. Unterlegscheiben 6 dienen zur Einstellung des Distanzstücks auf die erforderliche Länge. Das Distanzstück 1 enthält einen Längsschlitz 7, durch den die im Nagel 5 verschraubbare Schraube 8 hindurchragt. Durch Angriff an dieser Schraube oder anderen am Nagel vorgesehenen Angriffsmitteln läßt sich der Nagel 5 in Längsrichtung verschieben und in der gewünschten Stellung fixieren.

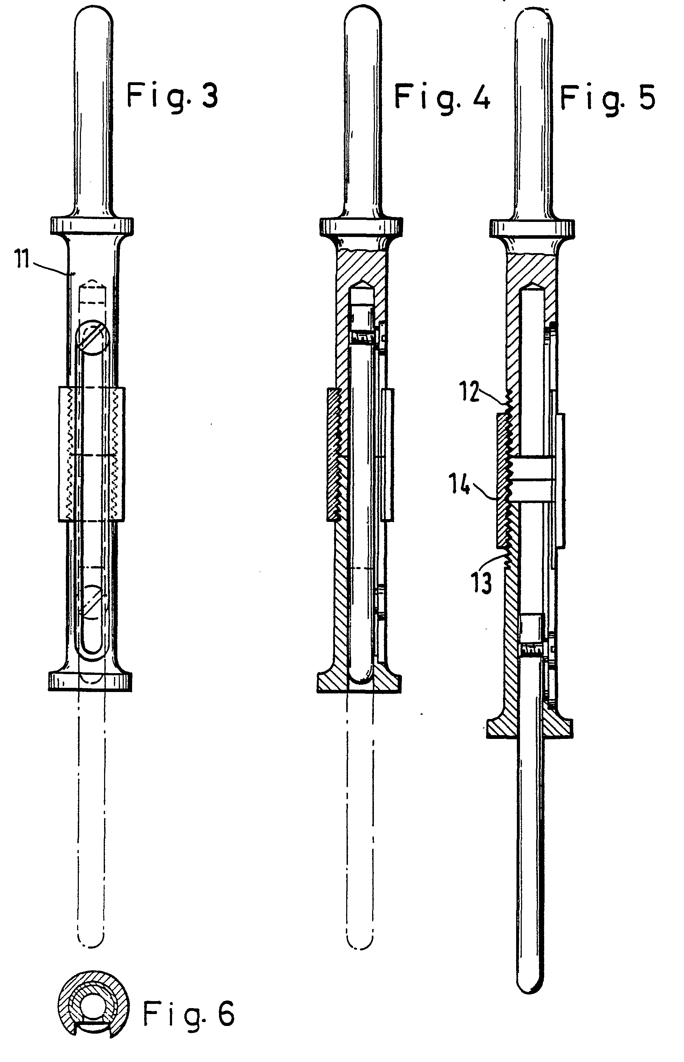

Die Ausführungsform gemäß Fig. 3 bis 6 stimmt, soweit im folgenden nichts anderes angegeben ist, mit derjenigen gemäß Fig. 1 und 2 überein. Das Distanzstück 11 ist in der Mitte geteilt und trägt an seinem Außenumfang einerseits Rechtsgewinde 12 und andererseits Linksgewinde 13, wobei die Distanzstückteile durch eine entsprechend mit Rechts- bzw. Linksgewinde versehene Hülse 14 zusammengehalten und auf eine beliebige Länge extendierbar sind.

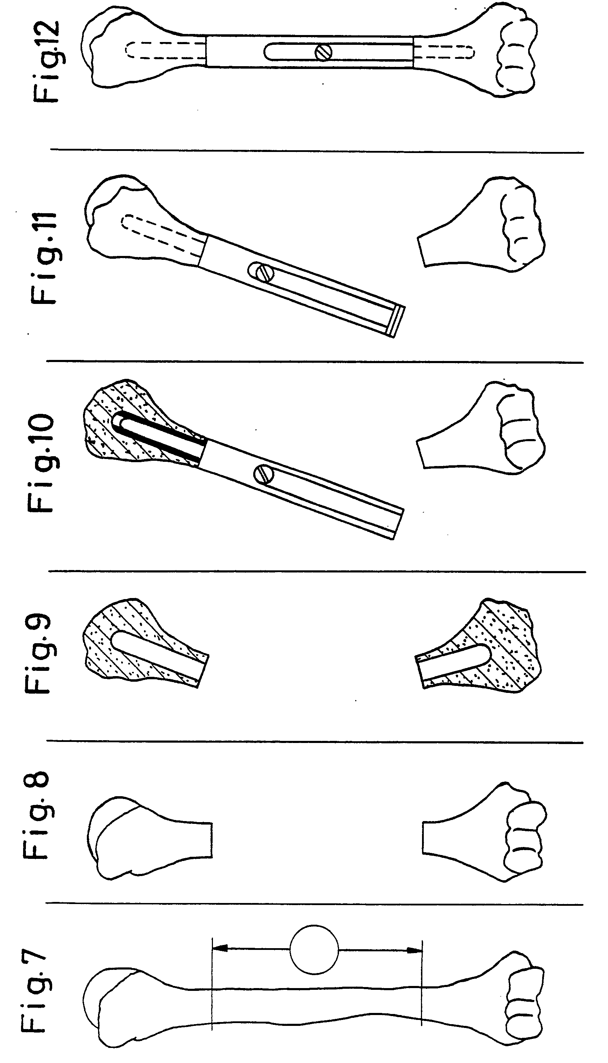

In Fig. 7 bis 12 erkennt man, daß zunächst das erforderliche Ersatzmaß 15 in situ gemessen wird. Nach der Resektion (Fig. 8) werden die Knochenenden gebeugt und ihr Markkanal zur Aufnahme der Nägel geöffnet. Gemäß Fig. 10 wird in das Knochenende 16 der fest mit dem Distanzstück 1 verbundene Nagel 4 eingeschoben. Die erforderliche Länge wird dann durch Unterlegscheiben 6 eingerichtet (Fig. 11). Danach werden die Teile in Streckstellung gebracht und wird der Nagel 5 durch Schlagen auf die Feststellschraube 8 in den Knochenteil 17 eingetrieben.

**Patentansprüche**

1. Endoprothese zum Ersatz des Mittelabschnitts eines langgestreckten Knochens, die aus einem hülsenförmigen Distanzstück (1) und einem darin längs verschiebbaren Nagel (5) zum Zusammenwirken mit einem Knochenendstück besteht, dadurch gekennzeichnet, daß der Nagel (5) einschließlich seines mit dem Knochenendstück zusammenzuwirken bestimmten Abschnitts in dem Distanzstück (1) aufnehmbar und von der Seite des Distanzstücks (1) her verschiebbar ist.

2. Endoprothese nach Anspruch 1, dadurch gekennzeichnet, daß ein Knochennagel (4) mit einem Ende des Distanzstücks (1) fest verbunden ist.

**Claims**

1. An endoprosthesis for the replacement of the medial portion of a long bone, which comprises a sleeve-like spacing member (1) and a pin (5) which is longitudinally displaceable therein and which serves to co-operate with a bone end piece, characterised in that the pin (5), including its portion intended to co-operate with the bone end piece, can be accommodated within the spacing member (1) and can be displaced from the side of the spacing member (1).

2. An endoprosthesis according to Claim 1, characterised in that a bone pin (4) is secured to one end of the spacing member (1).

**Revendications**

1. Endoprothèse pour le remplacement du segment intermédiaire d'un os long, se composant d'une pièce intercalaire en forme de douille (1) et d'une broche (5) qui peut coulisser longitudinalement dans celle-ci et qui est destinée à coopérer avec une épiphyse osseuse, caractérisée en ce que la broche (5) peut être reçue, y compris sa partie destinée à coopérer avec l'épiphyse osseuse, dans la pièce intercalaire (1) et en ce qu'on peut la faire coulisser par le côté de la pièce intercalaire (1).

2. Endoprothèse selon la revendication 1, caractérisée en ce qu'une broche osseuse (4) est fixée rigidement à l'une des extrémités de la pièce intercalaire (1).

Fig.1

Fig.2

Fig. 3

Fig. 4

Fig. 5

11

12

14

13

Fig. 6

Fig. 7  Fig. 8  Fig. 9  Fig. 10  Fig. 11  Fig. 12

3

EP 0 212 192 B1